# EUROPEAN PATENT APPLICATION

(11) **EP 4 714 443 A1**
(43) Date of publication of application: **25.03.2026**
(21) Application number: 24383003.1
(22) Date of filing: 19.09.2024
(51) Int. Cl.: A61K 31/416, A61P 9/10, A61P 25/28

(54) **HEME-REGULATED INHIBITOR KINASE ACTIVATORS FOR THE TREATMENT OF NEUROLOGICAL DISORDERS**

(71) Applicant: Fundación Pública Galega Instituto de Investigación Sanitaria de Santiago de Compostela, 15706 Santiago de Compostela A Coruña (ES); Servizo Galego De Saúde, 15703 Santiago de Compostela, A Coruna (ES); Universidade de Santiago de Compostela (USC), 15872 Santiago de Compostela A Coruña (ES); Centro de Investigación Biomédica en Red (CIBER), 28029 Madrid (ES)
(72) Inventor: SOBRINO MOREIRAS, Tomás, 15706 Santiago de Compostela (ES); ROMAUS SANJURJO, Daniel, 15706 Santiago de Compostela (ES); RODRÍGUEZ ARRIZABALAGA, Mariña, 15706 Santiago de Compostela (ES)
(74) Representative: Hoffmann Eitle

(57) **Abstract**

**METHOD FOR THE TREATMENT OF NEUROLOGICAL DISORDERS.** The present invention refers to Heme-Regulated Inhibitor (HRI) Kinase agonists or activators, or pharmaceutical composition comprising thereof, for use in a method for the treatment of a neurological disorder or for use as a neuroprotective agent.

## Description

### FIELD OF THE INVENTION

The present invention refers to the medical field. Particularly, the present invention refers to Heme-Regulated Inhibitor (HRI) Kinase agonists or activators, or pharmaceutical composition comprising thereof, for use in a method for the treatment of a neurological disorder or for use as a neuroprotective agent.

### STATE OF THE ART

The global burden of neurological disorders is increasing yearly worldwide, latest data showing that stroke and Alzheimer disease (AD) are the leading neurological causes of deaths by far: 67.4% and 20.3%, respectively.

Although each condition represents a different type of CNS damage (stroke: acute neuronal damage, AD: chronic neuronal damage), all two lead to the same cellular outcome: stress in the endoplasmic reticulum (ER) due to the accumulation of misfolded/unfolded proteins in the organelle. To counteract ER stress, two intertwined survival mechanisms are activated to restore balance: the unfolded protein response (UPR) and the integrated stress response (ISR). UPR detects aberrant proteins, and, among other mechanisms, this activates the PKR-like ER (PERK) kinase which triggers ISR by phosphorylating the eukaryotic translation initiation factor 2-alpha (p-eIF2α). This leads to a temporary block in the global synthesis of proteins and increases protein-folding and degradative capacities of the ER.

Unfortunately, there are no current reliable treatments promoting neuroprotection (stroke and AD), therefore, there is an unmet medical need to discover new targets and treatments that provide neuroprotection in damaged neurons after either of this CNS injuries.

The present invention is focused on solving this problem and an innovative therapeutic strategy is herein provided.

### DESCRIPTION OF THE INVENTION

### Brief description of the invention

The present invention refers to HRI Kinase agonists or activators, or pharmaceutical composition comprising thereof, for use in a method for the treatment of a neurological disorder or for use as a neuroprotective agent. Kindly note that the gene that encodes the HRI is known as EIF2AK1 (eukaryotic translation initiation factor 2 alpha kinase 1). This gene belongs to a family of kinases that phosphorylate the eukaryotic initiation factor 2 alpha (eIF2α), playing a key role in regulating protein synthesis in response to various cellular stresses, including heme deficiency. So, this patent application also cover the possibility of using agonists or activators of the gene EIF2AK1, not only HRI.

Particularly, preliminary *in vitro* data in primary cultured cortical mouse neurons, provided by the inventors of the present invention, encouragingly showed that the pharmacological blockage of the HRI kinase via the compound C27 (the most potent and specific inhibitor of HRI) radically diminished neuron viability without damage, which highlights the important role of the HRI kinase in the normal functioning of neurons. Curiously, the rs62456190 variant of the human HRI gene increased the age at onset of the X-linked dystonia-parkinsonism, a neurodegenerative disorder.

On the other hand, preliminary *in vitro* data also show that a temporary-guided activation of HRI by the HRI Kinase agonist or activator BTdCPU (1-(benzo[d][1,2,3]thiadiazol-6-yl)-3-(3,4-dichlorophenyl)urea) 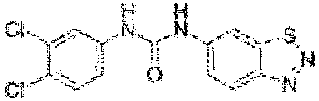 BTdCPU Chemical Structure CAS No.: 1257423-87-2 allows a longer UPR/ISR-like metabolic state that helps neurons to overcome cellular damage after either oxygen-glucose deprivation (OGD, *in vitro* stroke model) or Aβs exposure.

Remarkably, *in vivo* preliminary studies in a mouse model of ischemic stroke revealed that the BTdCPU dose of 10 mg/kg induced lower lesion volumes and best scores in motor analysis than controls.

Overall, these results support a beneficial impact of activating the HRI kinase following acute and chronic CNS damages.

As explained above, BTdCPU is used, in the context of the present invention, as a proof-of-concept HRI kinase agonist or activators. However, other N,N'-diarylureas derivatives could be used in the context of the present invention. N,N'-diarylureas are a class of organic compounds featuring two aryl groups attached to a urea group (-NH-CO-NH-). These chemical compounds are well established in the prior art, so they pertain to the common general knowledge. See for instance the reference [Chen T, Ozel D, Qiao Y, et al. Chemical genetics identify eIF2α kinase heme-regulated inhibitor as an anticancer target. Nat Chem Biol. 2011; 7(9):610-616. Published 2011 Jul 17. doi:10.1038/nchembio.613] which refers to N,N'-diarylureas like:
- 1-(2-chloro-5-nitrophenyl)-3- (3,4-dichlorophenyl)urea (NCPdCPU).
- 1-(benzo[d][1,2,3]thiadiazol-6-yl)-3-(3,4-dichlorophenyl)urea (BTdCPU).
- 1-(benzo[d][1,2,3]thiadiazol-6-yl)-3-(4-chloro-3-(trifluoromethyl)phenyl)urea (BTCtFPU).
- 1-(benzo[c][1,2,5]oxadiazol-5-yl)-3-(4-chlorophenyl)urea (BOCPU).

So, the first embodiment of the present invention refers to HRI Kinase agonist or activator, or pharmaceutical composition comprising thereof, for use in a method for the treatment of a neurological disorder or for use as a neuroprotective agent. The first embodiment also refers to a method for treating a neurological disorder that comprises the administration of a therapeutically effective dose or amount of an HRI Kinase agonist or activator.

In a preferred embedment of the present invention, the HRI Kinase agonist or activator is a N,N'-diarylureas derivative or salts derived thereof.

In a preferred embedment of the present invention, the HRI Kinase agonist or activator is BTdCPU or salts derived thereof.

In a preferred embedment of the present invention, the neurological disorder is Alzheimer disease or ischemic stroke.

The second embodiment of the present invention is an *in vitro* method for screening, identifying and/or producing compounds for use in a method for the treatment of a neurological disorder or as a neuroprotective agent which comprises: a) Assessing HRI Kinase activity once the candidate compound has been incubated with HRI Kinase, and b) wherein if an activation of HRI Kinase is observed, it is indicative that the candidate compound may be effective for the treatment of a neurological disorder or as a neuroprotective agent.

In a preferred embedment of the present invention, the present invention refers to an *in vitro* method for screening, identifying and/or producing compounds for use in a method for the treatment of Alzheimer disease or ischaemic stroke which comprises: a) Assessing HRI Kinase activity once the candidate compound has been incubated with HRI Kinase, and b) wherein if an activation of HRI Kinase is observed, it is indicative that the candidate compound may be effective for the treatment of Alzheimer disease.

For the purpose of the present invention the following terms are defined:
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of'. Thus, the phrase "consisting of" indicates that the listed elements are required or mandatory, and that no other elements may be present.
- By "therapeutically effective dose or amount" of a HRI Kinase agonist or activator is intended an amount that, when administered as described herein, brings about a positive therapeutic response in a subject having a neurological disease. The exact amount required will vary from subject to subject, depending on the species, age, and general condition of the subject, the severity of the condition being treated, mode of administration, and the like.

### Description of the figures

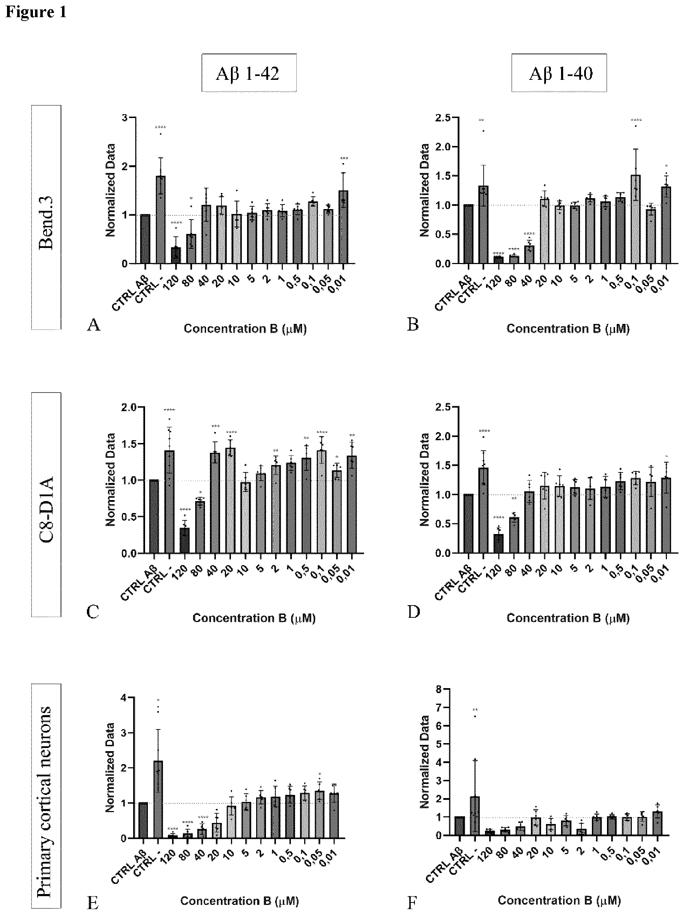

**Figure 1**. It shows that high concentrations of BTdCPU significantly diminished cell viability in all cell cultures exposed to both Aβ 1-40 and 42. However, concentrations of 0.1 and 0.01µM showed the highest level of cell viability in endothelial cells, astrocytes and neurons exposed to both Aβ 1-40 and 42.

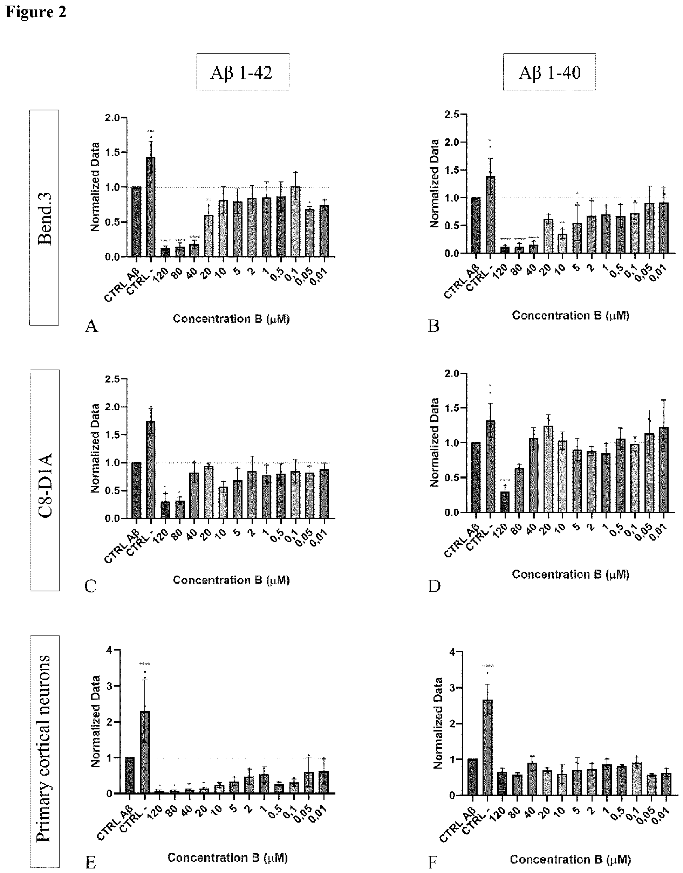

**Figure 2****.** It shows that BTdCPU-antagonist C27 compound confirms that previous results were due to a real beneficial role of BTdCPU on cells. To carry out this, C27 was added in the concentrations of BTdCPU used previously, and it was observed a general decreased of the viability in all cell types following both Aβ 1-40 and 42 exposures.

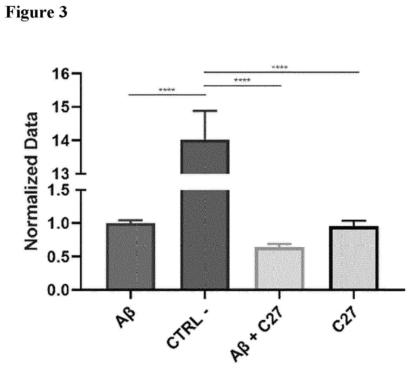

**Figure 3****.** It shows the same results as in **Figure 2** in cells no exposed to aggregates.

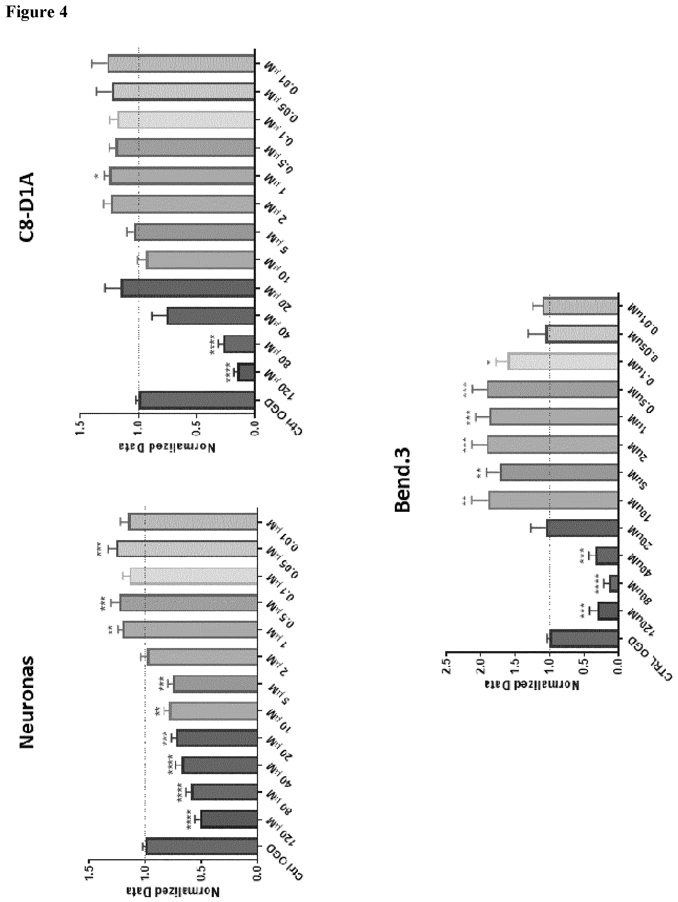

**Figure 4****.** Different concentrations of BTdCPU were used on different cell cultures to assess their effects following OGD. Concentrations below 1µM displayed lower viability than the control group. In general, 1µM was the concentration that showed the best level of cell viability in all cell types after OGD.

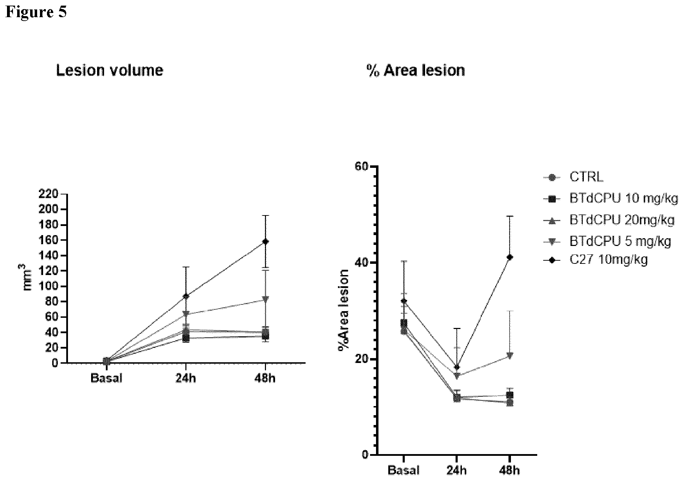

**Figure 5****.** Three different concentrations of BTdCPU: 5, 10 and 20 mg per kg of animal's weight were used. Moreover, a group of mice was treated with 10 mg/kg of C27. Preliminary results showed that 10 and 20 groups have similar lesion volumes and areas levels compared to control, whereas mice receiving 5 mg/kg of BTdCPU and 10 mg/kg of C27 have higher lesion volumes and areas.

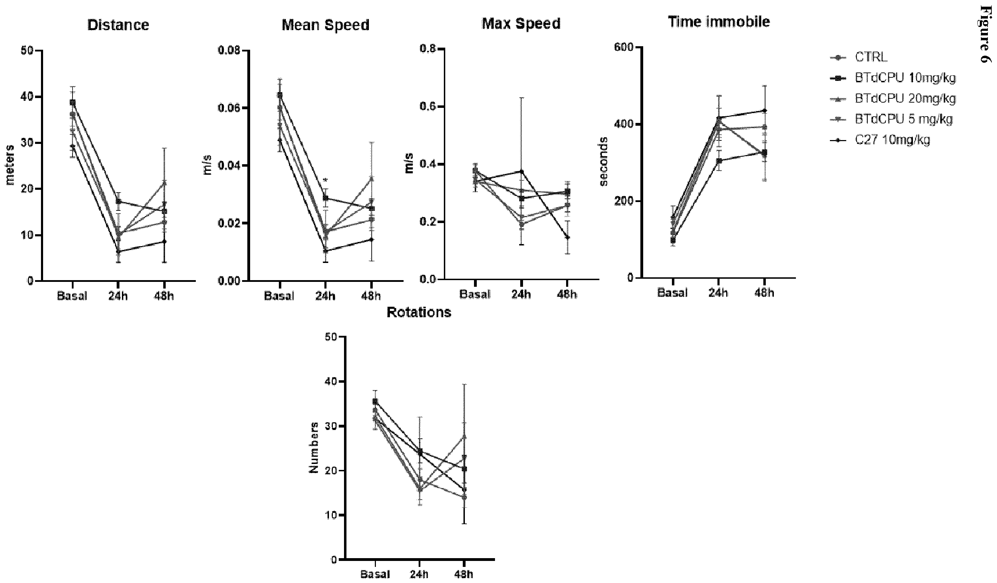

**Figure 6****.** The group treated with 10 mg/kg of BTdCPU showed the best scores in motor analysis. Specifically, they walked higher distances, had higher mean and maximum speed, were immobile less time, and displayed more body rotations.

### Detailed description of the invention

The present invention is illustrated by means of the Examples set below without the intention of limiting its scope of protection.

### Example 1. Material and Methods

### Example 1.1. Cell cultures

We used the murine endothelial cell line Bend.3 and the mouse cortical astrocytic cell line C8-D1A. Both cell types were allowed to growth until achieving a cellular confluence of approx. 90%.

Primary cortical neurons were performed from brain cortex of mouse E18 embryos. Neuronal cultures were used after 10 days of maturation.

### Example 1.2. Laboratory tests

Aβ 1-40 and 1-42 peptides were dissolved in DMSO at a concentration of 1mM, then each peptide were diluted in culture medium at 2µM and let in an incubator for 24h at 37°C to induce soluble aggregates. Following 24h, these protein aggregates were applied in cell cultures at 1µM concentration for 24h.

Cell cultures underwent oxygen-glucose deprivation (OGD) for 2h by changing medium to a glucose free one and placing them in a hypoxic environment, and then returned to a medium with glucose and incubator.

Cell viability was determined by measuring mitochondrial activity through MTT assay. These experiments were performed in cultures following either OGD or Aβ 1-40/42 exposure. Absorbency at 570nm was measured to indirectly assess viability.

### Example 1.3. Mouse model of ischemic stroke: transient middle cerebral artery occlusion (tMCAO)

The common carotid artery was exposed through a midline neck incision and dissected free of the surrounding nerves. A coated monofilament was inserted into the left external carotid artery, advanced into the internal carotid artery, and wedged into the cerebral arterial circle to obstruct the origin of the middle cerebral artery. After 50 min of surgery, the filament was withdrawn.

### Example 1.4. Drug treatments

The following drugs were used to treat the animals following tMCAO: BTdCPU (HRI kinase activator) and C27 (HRI kinase inhibitor), both diluted in DMSO. Four experimental groups were chosen: 1) Control, DMSO, n = 10; 2) BTdCPU 5 mg/kg, n = 4; 3) BTdCPU 10 mg/kg, n = 7; 4) BTdCPU 20 mg/kg, n = 7; 5) C27 10 mg/kg, n = 4. Mice underwent intraperitoneal injections of either DMSO or BTdCPU or C27 at 1 and 24 h following injury.

### Example 1.5. Magnetic resonance imaging (MRI)

The MRI studies were conducted on a 9.4-T horizontal bore magnet MRI system, Biospec 94/20USR (Bruker BioSpin, Bremen, Germany). Diffusion volumes were determined from diffusion-weighted image (DWI) maps, whereas infarct volumes were determined from T2 maps, both by manual selection. DWI images were used to determine the initial damage volume and ensure similar damage in all animals and were taken 40 minutes following artery occlusion. T2-weighted images were acquired at 24 and 48 hours after tMCAO.

### Example 1.6. Motor tests

The Any-Maze software was used to assess motor behavior of animals at different time points: baseline (before tMCAO) and 24- and 48-hours following surgery, prior to MRI. Different parameters were measured: distance traveled, mean and max speed, time immobile and rotations of the body.

### Example 2. Results

### Example 2.1. Assess the effect of BTdCPU on cell viability following Aβ 1-40/42 exposure

We used different concentrations of BTdCPU on different cell cultures to assess its effects on several cell types. Overall, we observed that high concentrations of BTdCPU significantly diminished cell viability in all cell cultures exposed to both Aβ 1-40 and 42. However, concentrations of 0.1 and 0.01µM showed the highest level of cell viability in endothelial cells, astrocytes and neurons exposed to both Aβ 1-40 and 42 **(****Figure 1****).**

Then, we used the BTdCPU-antagonist C27 compound to confirm that previous results were due to a real beneficial role of BTdCPU on cells. To carry out this, we added C27 in the concentrations of BTdCPU used previously, and we observed a general decreased of the viability in all cell types following both Aβ 1-40 and 42 exposures **(****Figure 2****).** Importantly, this was also observed in cells no exposed to aggregates **(****Figure 3****).**

### Example 2.2. Assess the effect of BTdCPU on cell viability following OGD

Similar to Aβ 1-40/42 experiments, we used different concentrations of BTdCPU on different cell cultures to assess its effects following OGD. Concentrations below 1µM displayed lower viability than the control group. In general, 1µM was the concentration that showed the best level of cell viability in all cell types after OGD **(****Figure 4****).**

### Example 2.3. Assess the effect of activating or inhibiting HRI kinase following tMCAO

Being aware of the difficulty in translating in vitro doses to in vivo studies, I decided to use three different concentrations of BTdCPU: 5, 10 and 20 mg per kg of animal's weight. Moreover, a group of mice was treated with 10 mg/kg of C27. Preliminary results showed that 10 and 20 groups have similar lesion volumes and areas levels compared to control, whereas mice receiving 5 mg/kg of BTdCPU and 10 mg/kg of C27 have higher lesion volumes and areas **(****Figure 5****).**

Importantly, the group treated with 10 mg/kg of BTdCPU showed the best scores in motor analysis. Specifically, they walked higher distances, had higher mean and maximum speed, were immobile less time, and displayed more body rotations **(****Figure 6****).**

In summary, these results suggest that 0.1 and 0.01µM concentrations are the most appropriate to administer BTdCPU in cells exposed to both Aβ 1-40 and 42. C27 experiments reveal that BTdCPU is mediating the beneficial effect seen in those cells affected by Aβ aggregates and also suggest an important role of this kinase in healthy cells. 1µM concentration of BTdCPU appears to be the most effective in increasing viability of endothelial cells, astrocytes and neurons following OGD. *In vivo* studies on a mouse model of ischemic stroke agree with *in vitro* results showing that a 10 mg/kg dose of BTdCPU offers the best outcomes in volume lesion and motor behavior. However, more animals are mandatory to complete the final n. Overall, these results support a beneficial impact of activating the HRI kinase following acute and chronic CNS damages.

## Claims

1. Heme-Regulated Inhibitor (HRI) Kinase agonist or activator, or pharmaceutical composition comprising thereof, for use in a method for the treatment of a neurological disorder or for use as a neuroprotective agent.

2. HRI Kinase agonist or activator, or pharmaceutical composition comprising thereof, for use, according to claim 1, **characterized in that** the HRI Kinase agonist or activator is a N,N'-diarylureas derivative.

3. BTdCPU, or pharmaceutical composition comprising thereof, according to any of the previous claims, for use in a method for the treatment of a neurological disorder or for use as a neuroprotective agent.

4. BTdCPU, or pharmaceutical composition comprising thereof, for use, according to any of the previous claims, in a method for the treatment of Alzheimer disease or ischemic stroke.

5. *In vitro* method for screening, identifying and/or producing compounds for use in a method for the treatment of a neurological disorder or as a neuroprotective agent which comprises: a) Assessing HRI Kinase activity once the candidate compound has been incubated with HRI Kinase, and b) wherein if an activation of HRI Kinase is observed, it is indicative that the candidate compound may be effective for the treatment of a neurological disorder or as a neuroprotective agent.

6. *In vitro* method, according to claim 5, for screening, identifying and/or producing compounds for use in a method for the treatment of Alzheimer disease or ischaemic stroke which comprises: a) Assessing HRI Kinase activity once the candidate compound has been incubated with HRI Kinase, and b) wherein if an activation of HRI Kinase is observed, it is indicative that the candidate compound may be effective for the treatment of Alzheimer disease.
